# EUROPEAN PATENT SPECIFICATION

(11) **EP 0 778 753 B1**
(45) Date of publication and mention of the grant of the patent: **22.09.1999**
(21) Application number: 94932170.7
(22) Date of filing: 01.11.1994
(51) Int. Cl.: A61F 2/04, A61L 27/00

(54) **METHOD OF MAKING AN ASYMMETRICAL POROUS PTFE FORM**
VERFAHREN ZU HERSTELLUNG EINER ASYMETRISCHEN PORÖSEN PTFE FORM
PROCEDE DE FABRICATION D'UNE FORME ASYMETRIQUE EN PTFE POREUX

(30) Priority: 02.09.1994 US 300306
(43) Date of publication of application: 18.06.1997
(73) Proprietor: W.L. GORE & ASSOCIATES, INC., Newark, Delaware 19714-9206 (US)
(72) Inventor: KASIC, James, F., Flagstaff, AZ 86004 (US); SIMMS, William, J., Flagstaff, AZ 86001 (US)
(74) Representative: Klunker . Schmitt-Nilson . Hirsch
(86) International application number: US9410607
(87) International publication number: WO9607370

(56) References cited:
- FR-A- 2 676 355
- US-A- 3 783 454
- US-A- 3 988 782
- US-A- 4 208 745
- US-A- 4 478 898
- US-A- 4 503 568
- US-A- 4 588 461
- US-A- 4 877 661
- US-A- 4 957 669

## Description

### FIELD OF INVENTION

This invention relates to a method of making asymmetrical porous PTFE forms and particularly to tapered, porous PTFE tubes useful as vascular grafts.

### BACKGROUND OF THE INVENTION

Porous polytetrafluoroethylene (hereinafter PTFE) forms have been known and used for some time. U.S. Patents 3,953,566 and 4,187,390 describe the manufacture of porous expanded PTFE forms such as tubes of substantially constant diameter wherein the porous expanded PTFE has a microstructure of nodes interconnected by fibrils. Tubes of this type subsequently found wide application, particularly as implantable vascular grafts. For some vascular graft applications, it was found desirable for the graft to be tapered between the opposing ends. Tapered porous PTFE tubes for use as vascular grafts (such as GORE-TEX® Vascular Grafts, Part Numbers V47020L and VT68110L, W. L. Gore & Associates, Inc., Flagstaff AZ) became available commercially. These tapered porous PTFE vascular grafts were made by methods that resulted in tubes having a wall thickness at the smaller diameter end of the tube that was greater than the wall thickness at the larger end of the tube. One such method is described by U.S. Patent 4,957,669 to Primm wherein a porous PTFE tube of substantially constant diameter is fitted over a tapered steel mandrel of round cross section to which ultrasonic energy is applied. The inside diameter of one end of the tube is increased with respect to the diameter of the opposite end by stretching the material of one end of the porous PTFE tube over the larger end of the mandrel. The result of such a stretching procedure is that the wall thickness of the larger diameter end of the tube is reduced, thereby becoming less than the wall thickness of the smaller diameter end of the tube. Because the larger diameter end of the tapered porous PTFE tube is made by stretching the smaller, original diameter thereby resulting in thinning of the wall thickness at the larger diameter end, it is seen that the original mass of the beginning smaller diameter end is maintained at the new, enlarged large diameter end. This can be confirmed by, for example, cutting ring-shaped samples of constant and equal width from each end of the tube with the samples cut perpendicular to the longitudinal axis of the tube. The large and small diameter, equal width samples can be expected to have substantially equal weights.

To have a thinner wall thickness at the larger diameter end of a tapered tube containing liquid under pressure is clearly undesirable from a mechanical standpoint in view of the greater hoop stress on the larger end of the tube. A tapered porous PTFE tube having at least equal wall thicknesses at the opposing ends would be mechanically preferable to the presently available tubes, and such a tapered tube having a greater wall thickness at the larger diameter end would be even more preferred. This would be particularly true for larger diameter vascular grafts such as about 12 mm diameter and larger intended for aortic repair.

### SUMMARY OF THE INVENTION

The present invention relates to a method of making asymmetrical porous PTFE forms wherein the porous PTFE form has at one end at least one dimension that is substantially different from the equivalent dimension of the opposite end.

According to the method of the present invention, an extrudate is created from a mixture of PTFE fine powder and liquid lubricant using a ram extruder, all in conventional manner. The symmetrical form of the extrudate thus created is then modified into an asymmetrical form by compression between two dies of the desired shape. Again according to convention, the liquid is removed from the asymmetrical extrudate which is then expanded by stretching at a temperature less than the crystalline melt temperature of PTFE, and finally and optionally, heat treated at a temperature greater than the crystalline melt temperature while being restrained to prevent shrinkage. The invention thus involves the additional step of modifying the extrudate to an asymmetrical form prior to removal of the liquid from the extrudate.

By asymmetrical is meant that one end of the form has at least one dimension that is substantially different from the equivalent dimension of the opposite end. These dimensions may relate to, for example, diameters or wall thicknesses of a tubular porous PTFE form. The form is not limited to that of a tube. A sheet extrudate may be modified with dies, dried and expanded by stretching to create a porous PTFE sheet having different thicknesses at opposing end. Alternatively, a tapered porous PTFE tube made according to the method of the present invention may be slit longitudinally to form a sheet having different thicknesses at opposing ends. Also, a tapered cylindrical form may be created having entirely different diameters at the opposing ends of the cylinder.

Also, a tapered porous PTFE tube may becreated having a wall thickness at the larger diameter end of the tube that is greater than or equal to the wall thickness at the smaller diameter end of the tube.

Tubes made according to the present invention are anticipated to be particularly useful as vascular grafts.

### BRIEF DESCRIPTION OF THE DRAWINGS

- Figure 1: describes a cross sectional view of a tapered porous PTFE tube of the prior art wherein the wall thickness of the large diameter end of the tube is less than the wall thickness of the small diameter end of the tube.
- Figure 2: describes a cross sectional view of a tapered tube made according to the method of the present invention wherein the wall thickness of the large diameter end of the tube is substantially equal to the wall thickness of the small diameter end of the tube.
- Figure 3: describes a cross sectional view of a tapered tube made according to the method of the present invention wherein the wall thickness of the large diameter end of the tube is substantially greater than the wall thickness of the small diameter end of the tube.
- Figures 4A, 4B and 4C: describe cross sectional views of the male and female dies used to modify constant diameter PTFE tubular wet extrudate to create the tapered porous PTFE tube made according to the method of the present invention.
- Figure 5: describes a cross sectional view of a porous PTFE tube having a constant inside diameter and a greater wall thickness at one end of the tube than at the opposite end.
- Figure 6: shows a tapered porous PTFE tube having a helical wrapping of porous PTFE film.
- Figure 7: describes a cross sectional view of a porous PTFE tube having substantially different wall thicknesses at different locations along the length of the tube.

### DETAILED DESCRIPTION OF THE INVENTION

Conventional porous PTFE tubes of substantially constant diameter and wall thickness are made by a process described by U.S. Patents 3,953,566 and 4,187,390. Briefly, this process involves blending fine powder PTFE resin with a lubricant, compressing the resulting mixture into a cylindrical pellet, extruding a tubular form from this pellet using a ram extruder, removing the lubricant from the tubular form, and then expanding the tube by stretching it parallel to its longitudinal axis after heating the tube to a temperature less than the crystalline melt point of PTFE. The resulting porous PTFE tube is then preferably heat-treated at a temperature above the crystalline melt point of PTFE while being restrained to prevent shrinkage. Porous PTFE tubing made by this process is of substantially constant diameter and wall thickness. Prior art, tapered tubes were made by, for example, increasing the diameter of one end of these constant diameter tubes by radial stretching, prior to the final step of heat-treating.

The tapered porous PTFE tube made according to the method of the present invention is made by using the above process through the step of extruding a tubular form. A step is then added to this process which requires taking the tubular extrudate which still contains the liquid lubricant and placing it between tapered male and female dies. The dies are preferably heated to a temperature such as about 60°C. The diametrical relationship between the male and female dies can be such that when fitted concentrically together, the space between the dies at the opposing larger and smaller diameter ends is equal, or alternatively the space at the larger diameter end is greater than the space at the smaller diameter end. Preferably the diameter of the extrudate should approximately equal the mean of the large and small diameters of the dies. Axial compression is applied to the two dies with the tubular extrudate between them. After releasing the compression, the tubular extrudate is removed from between the male and female dies. The removed tubular extrudate is tapered with wall thickness corresponding to the space between the dies. The liquid lubricant is then removed from the tapered tubular extrudate, which is subsequently expanded by stretching in a direction parallel to the longitudinal axis and preferably heat-treated after stretching as described previously.

As opposed to tapered porous PTFE tubes of the prior art, tubes made according to the method of the present invention have different masses at the opposite tube ends. Again, this can be confirmed by cutting equal width samples from each end of the papered tube perpendicular to the longitudinal axis of the tube whereby each sample takes the form of a constant width ring with both samples being of equal width. The large diameter sample can be expected to have a weight at least ten percent greater than that of the smaller diameter sample. In order to be considered as a tapered tube, the diameter of the small end of the tube is expected to be equal to no more than ninety percent of the diameter of the large end of the tube.

The tapered porous PTFE tube made according to the method of the present invention may be mechanically reinforced by providing the tube with a helically-wrapped reinforcement of, for example, porous PTFE film, thereby increasing the hoop strength of the tube. Porous PTFE tubes having this type of reinforcement are presently available as GORE-TEX Vascular Grafts in the form of both straight, constant diameter tubes and tapered tubes. Porous PTFE films suitable for this reinforcement are made as taught by U.S. Patents 3,953,566 and 4,187,390. Briefly, the preferred films for this application are porous PTFE films having a microstructure containing fibrils oriented substantially parallel to each other, that is oriented substantially in one direction. These films are then slit into long narrow tapes of, for example, 1.5 cm width with the orientation of the fibrils parallel to the length of the tape so that the greatest strength of the tape is along its length. This tape may then be helically wrapped about the outer surface of the tapered tube following the step of expanding the tube lengthwise by stretching and the additional step of fitting the tube over a tapered steel mandrel. Preferably, the edges of the tape should overlap during wrapping; the amount of overlap used will affect the thickness of the tape application and consequently the hoop strength of the finished tube. If desired, an increased amount of overlap may be used on the large diameter end of a tapered tube in order to better accommodate the greater hoop stress at that end of the tube. The step of heat-treating at a temperature above the crystalline melt point of the PTFE is performed after the film application while the tube is still on the steel mandrel. By heating the film above the crystalline melt temperature of PTFE it is caused to adhere to the underlying tapered porous PTFE tube.

The porous PTFE tube may also be provided with exterior reinforcement in the form of rings or spirals of a non-porous material such as fluorinated ethylene propylene applied to the exterior of the tube in the fashion of, for example, a Ringed GORE-TEX Vascular Graft (Part No. R05020100L).

The porous PTFE may be provided with rapid recovery properties as taught by U.S. Patent 4,877,661. For tapered tubes of porous PTFE, the compression step of the rapid recovery process should be performed on a tapered steel mandrel such that the amount of mandrel taper corresponds with the degree of taper of the porous PTFE tube while longitudinally compressed in the desired amount.

While the tapered porous PTFE tube made according to the method of the present invention is primarily useful for implantable vascular graft applications, it is anticipated to have other uses as well, including non-medical applications as well as other medical applications. It is believed that the tubing may be useful for, for example, preventing adhesions around intramammary coronary grafts if the tubing has a small fibril length such as about five microns. If provided with a relatively thin wall such as less than about 0.25 mm, the tapered tube may be useful as an intraluminal vascular graft for aneurismal repair. Such a graft may be secured in place by the use of, for example, expandable stents. A tapered porous PTFE tube may also be useful in industrial applications as a filter or component in a filter system where the chemically inert character of the PTFE is of advantage.

For purposes of describing tapered tubes, diameter refers to the inside diameter of the tube. Tube diameter is best determined by gently but snugly fitting the tube over a gradually tapered steel mandrel of circular cross section having indicated diameter graduations at appropriate intervals. Wall thickness is determined by measuring thickness at three equally spaced points around the circumference of the exposed tube end using calipers with flat surface jaws of at least 3 mm width to avoid crushing the porous PTFE. The wall thickness is the mean of the three measurements.

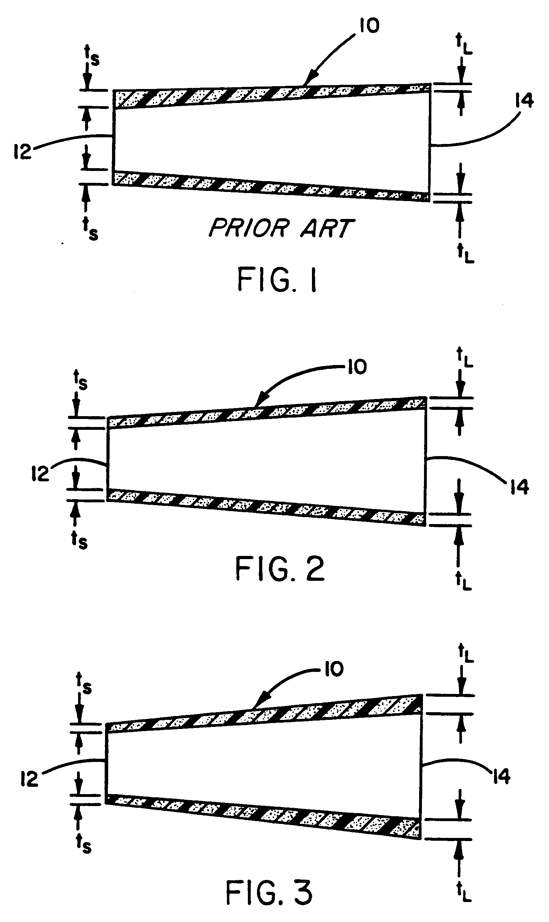

Figure 1 describes a cross sectional view of a tapered porous PTFE tube 10 of the prior art having a wall thickness t_{L} at the large diameter end 14 of the tube 10 that is less than the wall thickness t_{S} of the small diameter end 12. These are typically the result of modifying the diameter of one end of porous PTFE tubes of constant diameter that have been made by extruding a mixture of PTFE fine powder and a lubricant into a tubular form, removing the lubricant and expanding the tubular form by longitudinally stretching at a temperature less than the crystalline melt temperature of PTFE.

Figures 2 and 3 describe cross sectional views of porous PTFE tubes made according to the method of the present invention wherein the wall thickness t_{L} of the larger end 14 of the tube 10 is substantially equal to or greater than the wall thickness t_{S} of the smaller end 12 of the tube.

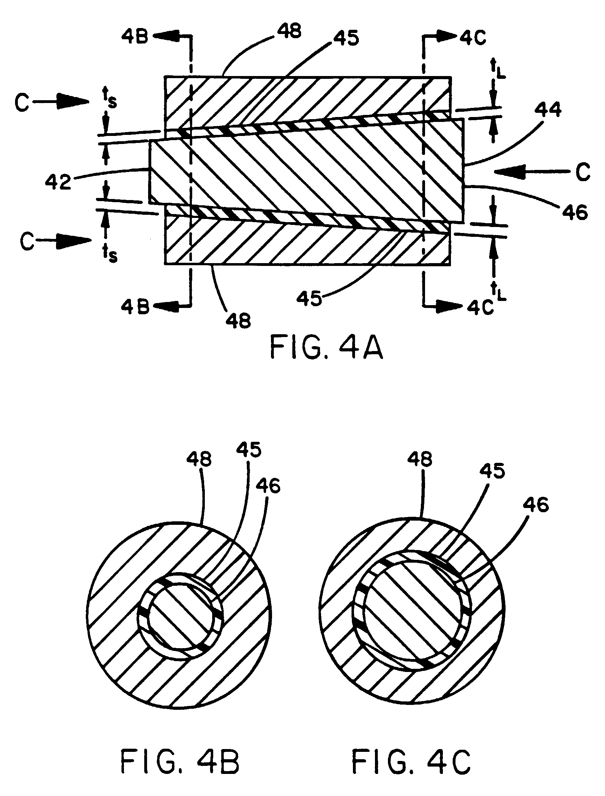

The inventive tube is made by extruding a tubular extrudate comprising a mixture of PTFE fine powder and lubricant, the extrudate having a substantially constant inside diameter and wall thickness. As shown by the cross sections of Figures 4A, 4B and 4C, the tubular extrudate 45, still containing liquid lubricant, is placed between tapered male die 46 and a tapered female die 48, with axial compression C applied to the resulting assembly. It is preferred that the dies be heated such as to about 60°C in order to aid in conforming the tubular extrudate to the shape of the dies.

Space t_{L} at the large end 44 of dies 46 and 48 and space t_{S} at the small end 42 are proportioned according to the desired wall thicknesses of the respective large and small ends of the finished porous PTFE tube. For a porous PTFE tube having large and small ends of equal wall thickness, the spaces t_{L} and t_{S} should be equal which requires that male die 46 and female die 48 have tapers of the same angles. If a porous PTFE tube is desired having a greater wall thickness at the larger end than at the smaller end, then space t_{L} at the large diameter end 44 of the dies 46 and 48 should be greater than space t_{S} at the smaller end 42 of dies 46 and 48. This necessitates that female die 48 should have a greater taper angle than male die 46.

After fitting the tubular extrudate containing liquid lubricant between male die 46 and female die 48, a compressive force represented by arrows C is applied to the assembly until spaces t_{S} and t_{L} are equal to the desired wall thicknesses of the finished porous PTFE tube. Finally, the compressive force is removed, the resulting tapered tubular form is removed from the dies and expanded by longitudinally stretching as taught by U.S. Patents 3,953,566 and 4,187,390. The resulting tapered porous PTFE tube may then be heat treated if desired.

While the transverse cross sections of Figures 4B and 4C indicate that the inventive form is transversely symmetrical, it is anticipated that the same method of modification of the shape of PTFE extrudate containing a liquid lubricant by the use of compressive dies to create a longitudinally asymmetrical shape may also be used to create a transversely asymmetrical shape if suitable dies are used.

Tapered porous PTFE tubes may be made by the method of the present invention with tapers ranging from very gradual to relatively severe. For example, a tapered tube useful as a vascular graft may have opposing ends with respective diameters of 6 mm and 8 mm, tapering gradually over a length of 100 cm. Alternatively, a tube of length as short as 10 cm may be made having a severe taper with opposing ends of, for example, 2 mm and 100 mm diameters. In still other embodiments, the tube may be provided with a non-linear, stepped taper by using dies having matched, but non-straight sided tapers. Additionally, the tube may be made with straight, constant diameter portions of both large and small diameters separated by a tapered section.

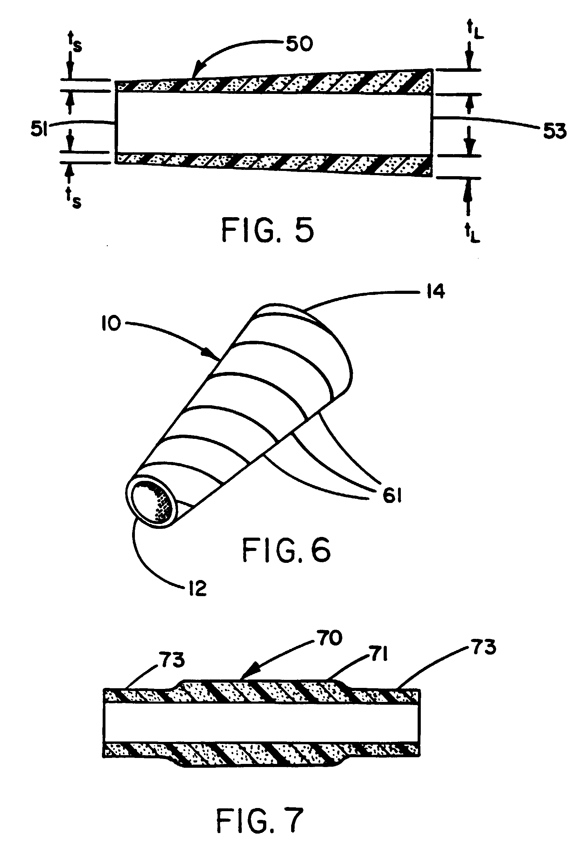

Figure 5 describes a cross sectional view of an asymmetrical porous PTFE tube 50 having a constant inside diameter and a greater wall thickness t_{L} at tube end 53 than the wall thickness t_{S} at tube end 51.

Figure 6 shows a tapered porous PTFE tube 10 having a helical wrapping 61 of a tape of porous PTFE film.

As shown by the cross section of Figure 7, it is also anticipated that a tube 70 may be made having a different wall thickness along the middle portion 71 of its length than at the ends 73. For example, a tubular vascular graft intended for arteriovenous access requiring frequent cannulation by dialysis needles can be expected to bleed less at the cannulation sites if the wall thickness of that middle portion of the length of the graft is of increased thickness. To make such a graft by the method of the present invention would require that the female die be split longitudinally to allow for its removal from the extrudate. The precursor extrudate should have a wall thickness equal to or slightly greater than the desired wall thickness of the thickest part of the tube. The outside diameter of the precursor extrudate should be equal to or very slightly greater than the outside diameter of the smallest outside diameter portion of the desired tube. The male die should be of the same outside diameter as the desired inside diameter of the finished tube, and should have a gradual taper at the first end to be inserted into the assembled split female die containing the precursor extrudate. The different wall thicknesses created by this method are continuous thicknesses, that is, are not the result of adding to the thickness by adding one or more layers of additional material to create extra thickness. It is apparent that these continuous, different wall thicknesses may have extra layers added such as reinforcing film which would not be included in determining thickness of the continuous, different wall thicknesses resulting from the method of the present invention.

### EXAMPLE 1

Fine powder PTFE resin was blended with 340 cm³ ISOPAR M, an odorless solvent (obtained from Exxon Corp., Ft. Allen, LA) per kg of the PTFE resin. The mixture was compressed into a tubular billet of 15.9 cm outside diameter, 3.8 cm inside diameter and 45.7 cm length, heated to about 40°C and extruded into tubes using a ram extruder having a reduction ratio of about 76:1. A 37 cm length of the resulting tubular extrudate, which had a wall thickness of 2.7 mm and inside diameter of 24 mm, was then placed between tapered male and female dies of circular cross section which had been heated to 60°C. The female die was of 36.8 cm length, with a bore of 56.92 mm at the large diameter end and a 17.32 mm bore at the small diameter end. The male die was of 42.65 cm length, with a large end diameter of 54.89 mm and a small end diameter of 14.27 mm. Both dies therefore incorporated a 3.06° taper measured from the longitudinal axis. Using a mechanical press, an axial compressive force of about 2500 kg was then applied to the assembly as shown by Figure 4A thus producing a tapered PTFE tube still containing the liquid lubricant. The lubricant was removed from the tapered PTFE tube by drying in forced convection air oven at 250°C for thirty minutes. The tube was then expanded by stretching longitudinally 6:1, at a rate of 55% per second in a forced convection air oven set at a temperature of about 310°C. The resulting tapered porous PTFE tube was subsequently heat treated in a gravity convection air oven set at about 393°C for 90 seconds.

### EXAMPLE 2

Another porous PTFE tube was created using the same method, materials and equipment of Example 1, except that the tapered male die was replaced by another male die having the same length but having a large end diameter of 55.83 mm and a small end diameter of 13.41 mm. The male die thus had a taper of 2.85°.

The porous PTFE tubes of Examples 1 and 2 were approximately 130 cm in length. The ends were cut off transversely from each of these tubes to create a tapered tube of 90 cm length. These tubes were then cut transversely into 30 cm lengths with the ends labeled 0 cm, 30 cm, 60 cm and 90 cm respectively beginning from the small diameter end. Inside diameter and wall thickness were measured at each tube end. A 1 cm wide ring-shaped sample was cut from an end adjacent to each labeled 30 cm interval and weighed. These data are shown in Table 1.

For comparison, the same measurements were made with a 4-7 mm tapered GORE-TEX Vascular Graft (Part No. V47050L) made by prior art methods. The 4 mm and 7 mm diameters of this graft are separated by a 5 cm long tapered section; measurements were made on constant diameter portions of the graft adjacent to the tapered section. At the 4 mm diameter end, the tube had an inside diameter of 3.8 mm, a wall thickness of 0.65 mm and the 1 cm wide ring-shaped sample weighed 0.064 g. At the 7 mm diameter end, the tube had an inside diameter of 6.8 mm, a wall thickness of 0.58 mm and the 1 cm wide ring-shaped sample weighed 0.061 g.

**TABLE 1**

| | EXAMPLE 1 | | | EXAMPLE 2 | | |
|---|---|---|---|---|---|---|
| Position | ID (mm) | WALL (mm) | MASS (g) | ID (mm) | WALL (mm) | MASS (g) |
| 0 cm | 19.50 | 1.19 | 0.52 | 14.57 | 0.11 | 0.02 |
| 30 cm | 26.10 | 1.21 | 0.61 | 16.37 | 0.20 | 0.02 |
| 60 cm | 32.60 | 1.19 | 0.77 | 19.36 | 0.59 | 0.11 |
| 90 cm | 38.35 | 1.15 | 1.06 | 26.97 | 0.79 | 0.69 |

## Claims

1. A method of making a tapered porous polytetrafluorethylene tube having a microstructure of nodes interconnected by fibrils and having first and second opposing ends wherein said first end has an inside diameter and a wall thickness and said second end has an inside diameter and a wail thickness, and wherein the inside diameter of said first end is less than ninety percent of the inside diameter of said second end and the wall thickness of said second end is greater than or equal to the wall thickness of said first end, said method comprising:
a) blending fine powder polytetrafluorethylene resin with a lubricant to create a mixture;
b) compressing the mixture into a pellet;
c) extruding a tubular form from the pellet;
d) axially compressing the tubular form between tapered male and female dies to create a tapered tubular form;
e) removing the tapered tubular form from between the tapered male and female dies;
f) removing the lubricant from the tapered tubular form; and
g) heating the tapered tubular form to a temperature less than the crystalline melt temperature of polytetrafluorethylene and expanding the tapered tubular form by stretching longitudinally, thereby creating the tapered porous polytetrafluorethylene tube.

2. A method according to claim 1 wherein the tapered porous polytetrafluorethylene tube is heated to a temperature greater than the crystalline melt temperature of porous polytetrafluorethylene while being restrained to prevent shrinkage.

3. A method according to claim 2 wherein the tapered porous PTFE tube is a vascular graft.

## Patentansprüche

1. Verfahren zur Herstellung einer sich verjüngenden porösen Polytetrafluorethylen-Röhre mit einer Mikrostruktur aus mittels Fibrillen verbundenen Knoten und mit einem ersten und einem zweiten entgegengesetzten Ende, bei der das erste Ende einen Innendurchmesser und eine Wandungsdicke bat und das zweite Ende einen Innendurchmesser und eine Wandungsdicke hat, und bei der der Innendurchmesser des ersten Endes weniger als 90% des Innendurchmessers des zweiten Endes beträgt und die Wandungsdicke des zweiten Endes größer oder gleich der Wandungsdicke des ersten Endes ist, wobei das Verfahren aufweist:
a) Vermengen von feinpulvrigem Polytetrafluorethylen-Harz mit einem Gleitmittel zur Erzeugung eines Gemisches;
b) Zusammenpressen des Gemisches zu einem Pellet;
c) Extrudieren eines röhrenförmigen Formteils aus dem Pellet;
d) axial Zusammenpressen des röhrenförmigen Formteils zwischen einer sich verjüngenden Patrize und einer sich verjüngenden Matrize zur Erzeugung eines sich verjüngenden röhrenförmigen Formteils;
e) Entfernen des sich verjüngenden röhrenförmigen Formteils aus zwischen der sich verjüngenden Patrize und der sich verjüngenden Matrize;
f) Entfernen des Gleitmittels von dem sich verjüngenden röhrenförmigen Formteil; und
g) Erwärmen des sich verjüngenden röhrenförmigen Formteils auf eine geringere Temperatur als die Kristallschmelztemperatur von Polytetrafluorethylen und Dehnen des sich verjüngenden röhrenförmigen Formteils durch Ziehen in Längsrichtung unter Erzeugung der sich verjüngenden porösen Polytetrafluorethylen-Röhre.

2. Verfahren nach Anspruch 1, bei dem die sich verjüngende poröse Polytetrafluorethylen-Röhre auf eine höhere Temperatur als die Kristallschmelztemperatur von porösem Polytetrafluorethylen erwärmt wird, während die festgehalten wird, um ein Schrumpfen zu verhindern.

3. Verfahren nach Anspruch 2, bei dem die sich verjüngende poröse PTFE-Röhre ein Gefäßtransplantat ist.

## Revendications

1. Procédé pour fabriquer un tube de polytétrafluoroéthylène poreux effilé ayant une microstructure de noeuds interconnectés par des fibrilles et ayant une première et une deuxième extrémités opposées dans lesquelles ladite première extrémité possède un diamètre interne et une épaisseur de paroi et ladite deuxième extrémité possède un diametre interne et une épaisseur de paroi, et dans lequel le diamètre interne de ladite première extrémité est inférieur à quatrevingt-dix pourcent du diamètre interne de ladite deuxième extrémité et l'épaisseur de la paroi de ladite deuxième extrémité est supérieure ou égale à l'épaisseur de la paroi de ladite première extrémité, ledit procédé consistant:
a) à mélanger une résine de polytétrafluoroéthylène en poudre fine avec un lubrifiant pour créer un mélange;
b) à comprimer le mélange en un granulé;
c) à extruder une forme tubulaire du granulé;
d) à comprimer axialement la forme tubulaire entre des matrices mâle et femelle effilées pour créer une forme tubulaire effilée;
e) à retirer la forme tubulaire effilée d'entre les matrices mâle et femelle effilées;
f) à retirer le lubrifiant de la forme tubulaire effilée; et
g) à chauffer la forme tubulaire effilée à une température inférieure à la température de fusion cristalline du polytétrafluoroéthylène et à allonger la forme tubulaire effilée en étirant longitudinalement, créant ainsi le tube de polytétrafluoroéthylène poreux effilé.

2. Procédé selon la revendication 1, dans lequel le tube de polytétrafluoroéthylène est chauffé à une température supérieure à la température de fusion cristalline du polytétrafluoroéthylène poreux tout en étant restreint pour empêcher un retrait.

3. Procédé selon la revendication 2, dans lequel le tube de PTFE poreux effilé est une greffe vasculaire.
